# DEMANDE DE BREVET EUROPEEN

(11) **EP 0 412 892 A1**
(43) Date de publication de la demande: **13.02.1991**
(21) Numéro de dépôt: 90402244.9
(22) Date de dépôt: 06.08.1990
(51) Int. Cl.: C07D 309/38, C07D 213/68

(54) **Nouvelle pyranone, procédé pour sa préparation, son application à la préparation d'une nouvelle pyridinone et son procédé de préparation**

(30) Priorité: 11.08.1989 FR 8910831
(71) Demandeur: ISOCHEM (société anonyme), F-75008 Paris (FR)
(72) Inventeur: Wirth, Didier, F-75007 Paris (FR); Gibert, Dominique, F-60340 Villers-S/Saint-Leu (FR); Ferrucio, Laurence, F-75019 Paris (FR)

(57) **Abrégé**

L'invention concerne la triméthyl-2,3,5-(4H) pyranone,de formule : 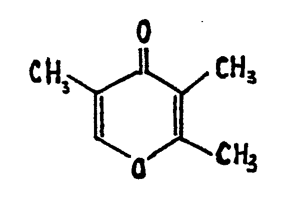 que l'on prépare par acétylation d'une cétone dérivée de la formyl-2-pentanone-3.

Application à la preparation de la triméthyl-2,3,5-(1H) pyridinone-4 de formule: 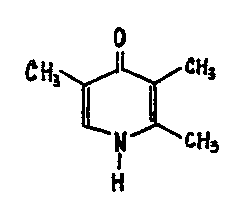 par ammonolyse de la triméthyl-2,3,5-(4H) pyranone-4.

## Description

La présente invention se rapporte à la triméthyl-2,3,5-(4H) pyranone-4, à un procédé pour sa préparation, à son applica- à la préparation de la triméthyl-2,3,5-(1H) pyridinone-4, ainsi qu'à un procédé de préparation de la triméthyl-2,3,5-(1H) pyridinone-4 par l'intermédiaire de la triméthyl-2,3,5-(4H) pyranone-4.

La présente invention a donc pour objet la triméthyl-2,3,5-(4H) pyranone-4, ainsi qu'un procédé permettant de la préparer dans de bonnes conditions et avec un bon rendement.

L'invention a également pour objet l'application de la triméthyl-2,3,5-(4H) pyranone-4 à la préparation de la triméthyl-2,3,5-(1H) par ammonolyse.

La présente invention a enfin pour objet un procédé de synthèse industrielle de la triméthyl-2,3,5-(1H) pyridinone-4, corps nouveau, utile comme intermédiaire de fabrication de produits pharmaceutiques.

Plusieurs agents antiulcéreux, tels que l'Oméprazole, comportent dans leur formule un motif dérivé d'une alcoxy-4-triméthyl-2,3,5-pyridine (I) : 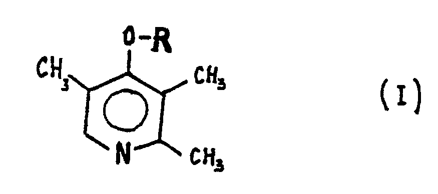 formule dans laquelle R désigne un groupe alkyle comportant de 1 à 3 atomes de carbonel éventuellement substitué par un ou plusieurs halogènes.

La synthèse classique de ces intermédiaires fait appel à la nitration du N-oxyde de triméthylpyridine opération réputée dangereuse (voir par exemple : Chem. & Eng. News, 30 janvier 1989 p.2).

On peut considérer que les alcoxy-4-triméthyl-2,3,5-pyridines de formule générale (I) sont des dérivés fonctionnels du triméthyl-2,3,5 pyridinol-4 ou triméthyl-2,3,5(1H) pyridinone-4, formes tautomères suivant la formule (II) : 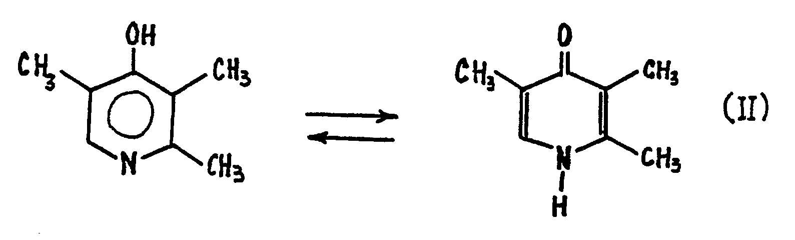 Assez curieusement,ce corps est absent de la littérature chimique et la présente invention offre précisément une synthèse industrielle de ce nouvel intermédiaire, simple et sans danger.

Selon la présente invention, on prépare la triméthyl-2,3,5-(1H) pyridinone-4 en deux étapes à partir d'un dérivé de la formyl-2-pentanone-3 de formule générale (III) : 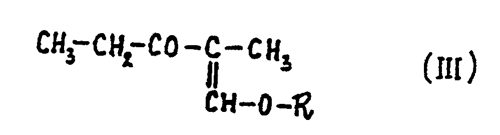 dans laquelle R désigne un atome d'hydrogène, un atome de métal alcalin, un radical alcoyle comportant de 1 à 8 atomes de carbone ou encore un radical acétyle, l'un ou l'autre éventuellement substitué par un ou plusieurs halogènes.

Dans un premier temps, l'action d'un agent acétylant sur les cétones de formule générale (III) conduit à la triméthyl-2,3,5-(4H) pyranone de formule (IV) : 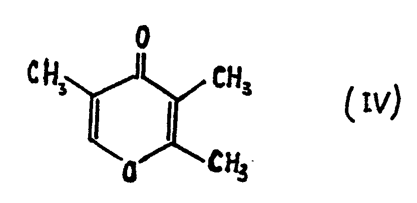 corps nouveau entrant dans le cadre de la présente invention.

Dans un second temps, l'action de l'ammoniac sur la pyranone de formule (IV) permet l'obtention quasi quantitative de la pyridinone de formule (II) recherchée: 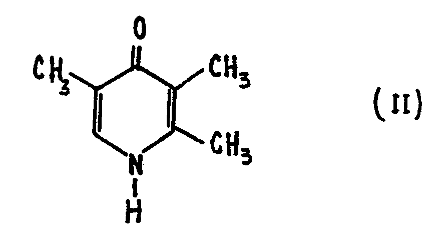 que l'homme de l'art saura transformer à son gré en l'intermédiaire de son choix: chloro triméthyl pyridine, méthoxy triméthyl pyridine ou autres.

Les produits de départ, qui correspondent à la formule générale (III), sont connus et aisément accessibles (voir par exemple: K.C. BRANNOK, J.A.C.S. 75 p. 2050 et R.D. SHRIVASTAVA, J. Ind. Chem. Soc. 1978 p. 1273). On les obtient généralement par formylation de la diétyl cétone à l'aide d'un formiate d'alcoyle tel que le formiate de méthyle ou le formiate d'éthyle, en présence d'un alcoolate alcalin tel que le méthylate de sodium ou de potassium, ou encore l'éthylate de sodium.

Les sels deformyl-2-pentanone-3 ainsi obtenus peuvent ensuite être convertis en formyl-2-pentanone-3 libre par acidification, ou en éthers d'enols de celle-ci paraction d'un alcool en milieu acide anhydre ou par action d'un agent alcoylant tel que les sulfates de méthyle ou éthyle et les halogènures tels que le chlorure, le bromure ou l'iodure de méthyle, éthyle, isopropyle ou autres.

L'action d'un agent acétylant tel que le chlorure d'acétyle, l'anhydride acétique ou l'acétate d'isopropényle, permet d'accéder au dérivé répondant à la formule générale (III) pour lequel R est un radical acétyle.

L'acétylation des cétones répondant à la formule générale (III), nouvelle, peut être observée lors de l'action d'un agent tel que l'anhydride acétique, le chlorure d'acétyle ou le bromure d'acétyle en présence d'un acide de Lewis tel que le trifluorure de bore ou des halogénures d'aluminium,de cobalt ou de zinc ou bien encored'une base telle que les amidures ou alcoolates alcalins. On opère de préférence à température basse ou modérée, comprise entre -40°C et +30°C.

La triméthyl-2,3,5-(4H) pyranone-4 est isolable après reprise à l'eau du mélange réactionnel et purifiable par distillation ou recristallisation dans un solvant tel que l'hexane ou l'heptane.

L'ammonolyse finale est menée de préférence entre 100°C et 150°C sous une pression d'ammoniac de 5 à 15 bars, par exemple en milieu aqueux ou hydroalcoolique. La triméthyl pyridone, peu soluble dans les solvants non protiques, cristallise aisément.

Les exemples suivants, donnés à titre indicatif, d'une forme de réalisation, illustreront davantage l'invention, sans en limiter la portée pour autant:

### Exemple 1 : Triméthyl-2,3,5-(4H) pyranone-4

On fait absorber 48g de trifluorure de bore par un mélange de 36 g d'acide acétique et 15,5 g d'anhydride acétique, puis on coule sur la suspension ainsi obtenue un mélange de 15,5 g d'anhydride acétique et 19,2 g de méthoxy-1-méthyl-2-pentène-1-one-3. Après 3 jours d'agitation à 25°C, puis hydrolyse suivie d'extraction au chlorure de méthylène, lavage jusqu'à neutralité et concentration de la phase organique, on obtient 7,7 g de triméthyl pyranone brute distillant vers 80°C sous 1 mm de mercure.

Après recristallisation dans l'heptane le produit fond à 34°C.
Son spectre de RMN du proton (CDCl3) laisse apparaître 4 signaux:
3 H à δ = 1,91 ppm (doublet)
3 H à δ = 1,95 ppm (singulet)
3 H à δ = 2,27 ppm (singulet)
1 H à δ = 7,59 ppm (multiplet)

### Exemple 2 : Triméthyl-2,3,5-(1H) pyridinone-4

Dans un autoclave en acier inoxydable, on charge 1 partie en poids de triméthyl pyranone obtenue selon l'exemple 1 et 10 parties en poids de solution aqueuse d'ammoniac à 20%, puis on porte à 125°C pendant 24 heures : la pression atteint 14,5 bars.

Après refroidissement et concentration à sec, puis reprise du résidu à l'acétone et filtration, on obtient la pyridone recherchée avec un rendement de 92%.
-Point de fusion : 217°C
-Spectre de RMN du proton (DMSO D 6) :
6 H δ = 1,825 ppm (2 singulets)
3 H δ = 2,50 ppm (1 singulet)
1 H δ = 7,41 ppm (1 singulet)
1 H δ = 11,1 ppm (très large)

### Exemple 3 : Acétoxy-1-méthyl-2-pentène-1-one-3

On porte au reflux un mélange de 11,4 g de formyl-2-pentanone-3 et 17 g d'acétate d'isopropényle, en présence de 0,1 g d'acide sulfurique.Après élimination de l'acétone formée à pression ambiante, on distille sous vide le produit recherché. On obtient ainsi 11 g d'acétoxy-1-méthyl-2-pentène-1-one-3, passant à 104°C sous 16 mm de mercure, soit un rendement de 70%.
-Spectre de RMN du proton (CDCl3) :
3 H vers δ = 1,028 ppm (triplet)
3 H vers δ = 1,73 ppm (doublet)
3 H vers δ = 2,16 ppm (doublet)
2 H vers δ = 2,6 ppm (quadruplet)
1 H vers δ = 8,14 ppm (doublet)

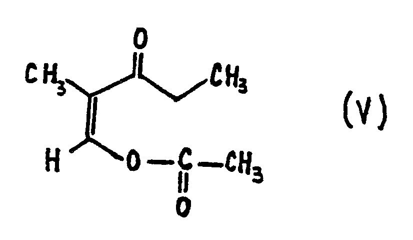

## Revendications

1 - Triméthyl-2,3,5-(4H) pyranone-4, de formule :

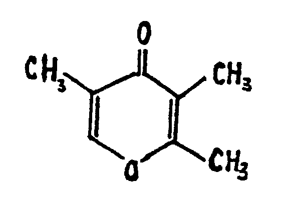

2 - Procédé pour la préparation du composé nouveau selon la revendication 1,caractérisé en ce que l'on effectue une réaction d'acétylation sur une cétone dérivée de la formyl-2-pentanone-3, de formule générale : 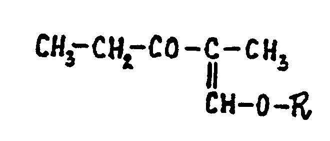 dans laquelle R désigne un atome d'hydrogène, un atome de métal alcalin, un radical alcoyle comportant de 1 à 8 atomes de carbone ou encore un radical acétyle, l'un ou l'autre éventuellement substitué par un ou plusieurs halogènes;
à l'aide d'anhydride acétique, de chlorure ou de bromure d'acétyle, à température comprise entre -40°C et +30°C.

3 - Procédé pour la préparation de la triméthyl-2,3,5-(4H) pyranone-4 selon la revendication 2, caractérisé par le fait que la réaction d'acétylation est effectuée en présence de trifluorure de bore ou des halogénures d'aluminium, de cobalt ou de zinc.

4 - Procédé pour la préparation de la triméthyl-2,3,5-(4H) pyranone-4 selon la revendication 2, caractérisé en ce que la réaction d'acétylation est effectuée sur l'acétoxy-1-méthyl-2-pentène-1-one-3.

5 - Acétoxy-1-méthyl-2-pentène-1-one-3,de formule : 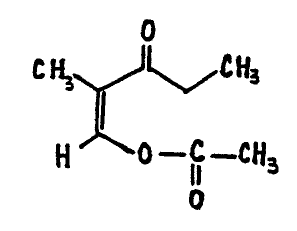 en tant qu'intermédiaire dans la preparation du composé selon la revendication 1.

6 - Application de la triméthyl-2,3,5-(4H) pyranone-4 selon la revendication 1 à la préparation de la triméthyl-2,3,5-(1H)pyridinone-4, de formule : 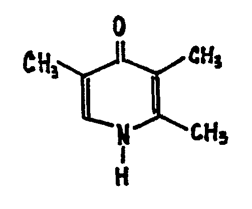 caractérisée en ce que l'on effectue l'ammonolyse de la triméthyl-2,3,5-(4H) pyranone-4, de préférence entre 100°C et 150°C, sous une pression d'ammoniac de 5 à 15 bars, en milieu aqueux ou hydroalcoolique.

7 - Triméthyl-2,3,5-(1H) pyridinone-4, de formule:

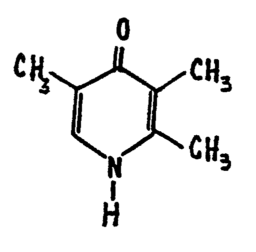

8 - Procédé de préparation de la triméthyl-2,3,5-(1H) pyridinone-4 caractérisé par la suite des réactions suivantes:
a) acétylation d'une cétone dérivée de la formyl-2-pentanone-3 de formule générale : 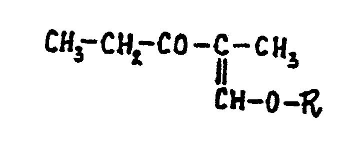 ou R a la même signification que dans la revendication 2, à l'aide d'anhydride acétique, de chlorure ou de bromure d'acétyle, à température comprise entre -40°C et + 30°C;
b) ammonolyse de la triméthyl-2,3 5-(4H) pyranone-4 obtenue en a)
